# DEMANDE DE BREVET EUROPEEN

(11) **EP 0 956 834 A2**
(43) Date de publication de la demande: **17.11.1999**
(21) Numéro de dépôt: 99401046.0
(22) Date de dépôt: 29.04.1999
(51) Int. Cl.: A61F 2/14, A61B 17/28

(54) **Kératoprothèse et outil pour le traitement de la partie haptique de celle-ci**

(30) Priorité: 15.05.1998 FR 9806150
(71) Demandeur: F.C.I. (FRANCE CHIRURGIE INSTRUMENTATION), F-92130 Issy Les Moulineaux (FR)
(72) Inventeur: Legeais, Jean-Marc, 94160 Saint-Mande (FR); Drubaix, Isabelle, 62520 Le Touquet (FR)
(74) Mandataire: Faber, Jean-Paul

(57) **Abrégé**

Kératoprothèse comprenant une partie optique transparente (1) formant lentille convergente et une partie haptique (2) de liaison avec le stroma cornéen et constituée par une couronne de polymère colonisable par les cellules cornéennes et surmoulée par son bord interne à la partie optique, caractérisée en ce que la partie haptique est pré-perméabilisée par immersion dans un liquide dont la tension de surface est de l'ordre de 20 dynes/cm, puis par immersion dans un liquide contenant au moins une macro-molécule naturellement présente dans le sang.

## Description

La présente invention vise une kératoprothèse, ou cornée artificielle, et un outil destiné au traitement de la partie haptique de cette dernière.

L'invention se rapporte à une kératoprothèse destinée à être implantée dans la cornée après trépanation de la zone centrale de celle-ci et comprenant une partie optique centrale et une partie haptique ou de support ayant la forme d'une couronne entourant la partie optique, et constituée en un matériau alloplastique biocompatible synthétique poreux colonisable par des tissus cornéens.

De tels implants sont connus et utilisés dans les cas où la cornée a perdu sa transparence à la suite d'un contact avec des composés caustiques, de chocs traumatiques, de brûlures, de trachomes, de pemphigoïdes, de syndromes de Lyell, etc..., et plus généralement, dans tous les cas où une greffe de cornée n'est pas possible. On découpe alors la zone centrale de la cornée par trépanation et on remplace ladite zone par une méso ou kératoprothèse.

La partie optique est généralement constituée par un cylindre transparent en PMMA (polyméthylemétacrylate), mais les parties haptiques diffèrent selon les fabricants ou les auteurs. Les parties haptiques ont pour but d'établir la fixation sur la cornée, l'épaisseur de celle-ci étant de l'ordre de 0,6 mm.

Le brevet français FR-A-2 608 041 propose de coller, autour du cylindre de PMMA, un tissu de "Dacron" (marque déposée) ou de polytétrafluoroéthylène.

Le brevet français FR-A-2 665 356 se rapporte à une cornée artificielle comprenant une optique transparente, incluse dans un orifice ménagé dans la cornée par trépanation, comprenant une pièce de fixation portant contre la surface externe de la cornée et une pièce support vissée sur l'optique portant contre la paroi interne de la cornée.

Les prothèses connues présentent différents inconvénients qui résultent notamment de ce que la partie optique est rigide et, par suite, ne permet pas la mesure de la tension interne du globe oculaire, paramètre essentiel pour le dépistage et le suivi clinique des glaucomes. Par ailleurs, l'optique cylindrique conduit à une prothèse épaisse et non ergonomique. Enfin, la jonction optique-partie haptique n'est, en général, pas suffisamment solide.

Pour remédier à ces inconvénients, il a déjà été proposé de constituer la partie optique à partir d'un matériau souple et transparent tel que du silicone ou un matériau hydrogel biocompatible. La souplesse de l'optique permet la mesure de la tension oculaire tandis que sa forme ergonomique s'intègre parfaitement dans la cornée réceptrice.

La kératoprothèse, selon l'invention, est du type comprenant une partie optique transparente formant lentille convergente et une partie haptique de liaison avec le stroma cornéen et constituée par une couronne de polymère colonisable par les cellules cornéennes et surmoulée, par son bord interne, à la partie optique et est caractérisée en ce que la partie haptique est pré-perméabilisée par immersion dans un liquide dont la tension de surface est de l'ordre de 20 dynes/cm puis par immersion dans un liquide contenant au moins une macro-molécule naturellement présente dans le sang.

La macro-molécule présente dans le sang peut être le sérum du sang du patient ou son sang lui-même.

Grâce à cette disposition, les pores de la partie haptique contiennent des macro-molécules (protéines, lipides, glucides, facteurs de croissance, cytokines, etc...) qui favorisent la migration, puis la multiplication des cellules concernées dans le polymère afin que, dès l'implantation, on assiste à la biointégration de celui-ci dans le stroma cornéen.

De préférence, le bord de la lentille est aminci pour présenter une solution de continuité entre l'optique et la partie haptique.

Ainsi, lors de l'implantation, on pratique une ouverture dans la cornée ayant un diamètre inférieur à celui de la prothèse de manière que ladite cornée recouvre le bord de l'optique.

Suivant une autre caractéristique, au moins, l'une des faces de l'optique est revêtue de polyvinyle pyrrolidone ce qui rend la prothèse plus agréable à supporter pour le patient et évite tout dépôt sur la surface de l'optique.

L'invention vise également un outil pour le traitement de la partie haptique avant implantation, cet outil comprenant deux branches solidaires l'une de l'autre à l'une de leurs extrémités, et dont les autres extrémités comportent chacune une coupelle présentant une partie concave, les concavités étant tournées en regard l'une de l'autre, ledit outil pouvant occuper une position d'ouverture dans laquelle les coupelles sont écartées l'une de l'autre de manière à pouvoir insérer entre celles-ci la lentille et une position de fermeture dans laquelle le bord des coupelles enserre la partie haptique au voisinage du bord de la lentille, ledit outil étant caractérisé en ce qu'il comporte une vis traversant les branches en un point intermédiaire de leur longueur et recevant un écrou.

On réalise ainsi un outil très simple qui permet, durant le traitement de pré-perméabilisation de la partie haptique, de protéger la lentille.

L'invention va maintenant être décrite avec plus de détails, en se référant à un mode de réalisation particulier, donné à titre d'exemple seulement, et représenté aux dessins annexés dans lesquels :
Figure 1 est une vue schématique en coupe d'une kératoprothèse selon l'invention, après implantation
Figure 2 est une vue partielle à plus grande échelle de la kératoprothèse
Figure 3 est une vue en élévation de l'outil
Figure 4 montre, à plus grande échelle, un détail de l'outil.

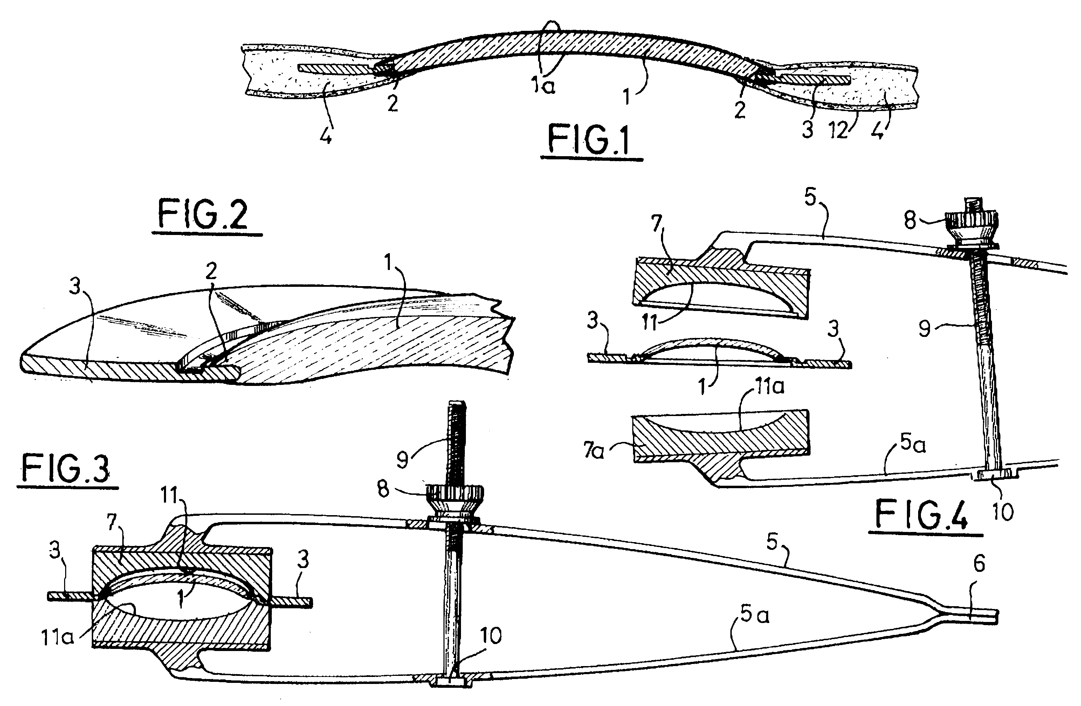

Aux figures 1 et 2, on a représenté une kératoprothèse comprenant une optique transparente formant lentille convergente 1 dont les bords 2 sont légèrement amincis et qui est surmoulée sur la surface interne d'une couronne de polytétrafluoroéthylène expansé (PTFEe), constituant la partie haptique 3.

La lentille 1 est en silicone, ses deux faces sont revêtues de polyvinyle pyrrolidone la. La jonction entre l'optique et la partie haptique résulte d'une pénétration intime du silicone dans les pores du PTFEe.

Cette pénétration est rendue possible grâce au caractère très hydrophobe des deux matériaux. Cette jonction, véritable imbrication des deux matériaux l'un dans l'autre, s'est révélée plus résistante que les jonctions précédemment décrites. L'absence de discontinuités entre l'optique et la partie haptique améliore notablement la stabilité à long terme de la kératoprothèse en facilitant la réépithalisation de l'optique (recouvrement de celle-ci par l'épithélium cornéen).

Mais le PTFEe est naturellement hydrophobe et cette caractéristique n'est pas favorable à une colonisation par les cellules cornéennes ; l'invention prévoit un traitement pré-opératoire de cette partie haptique afin d'améliorer la biointégration et permettre une colonisation rapide.

Aux figures 3 et 4, on a représenté un outil facilitant le traitement pré-opératoire de la partie haptique et présentant deux branches élastiques 5, 5a dont les extrémités 6 sont solidaires l'une de l'autre.

Chaque branche 5, 5a, est solidaire, à son extrémité libre, d'une coupelle 7, 7a, présentant un logement concave 11, lla. Les logements 11, lla, sont tournés l'un en regard de l'autre.

Les branches 5, 5a, étant élastiques, elles tendent normalement à s'écarter de manière que les coupelles puissent être éloignées l'une de l'autre.

Les deux branches 5, 5a, sont traversées par une vis 9 présentant une tête 10 coopérant avec la surface externe de la branche 5a, tandis que l'extrémité libre de la vis 9 reçoit un écrou 8.

Ainsi, en dévissant l'écrou 8, on peut permettre l'ouverture pour loger, entre les coupelles 7, 7a, l'optique 1 de la kératoprothèse, puis, en vissant l'écrou 8, enserrer entre les bords des logements 11 la partie haptique 3, juste au voisinage du bord de la lentille 1. Ainsi la partie haptique 3 fait saillie à l'extérieur des coupelles 7, 7a.

On procède ensuite à un traitement de la partie haptique par immersion dans un liquide présentant une tension de surface de l'ordre de 20 dynes/cm, tel que de l'éthanol. Le PTFEe, préalablement blanc opaque et hydrophobe, devient instantanément translucide et perméable. Néanmoins, le caractère hydrophobe du polymère en PTFEe réapparaît dès l'évaporation de l'éthanol ou autre liquide de tension de surface équivalent. L'immersion de la prothèse, après le passage dans l'éthanol, dans un bain de sérum ou de sang autologue (prélevé sur le patient), ou dans une substance contenue dans le sang (d'origine humaine, animale ou synthétique) permet de maintenir durablement la translucidité et le caractère hydrophile de la partie haptique. La kératoprothèse ainsi traitée est rincée deux fois avec du sérum physiologique ou "BSS" (marque déposée), afin d'éliminer toute trace d'éthanol.

A l'issue de ce traitement, avant implantation de la prothèse, les pores de la partie haptique contiennent des macromolécules (protéines, lipides, glucides, facteurs de croissance, cytokines, etc...) favorisant la migration, puis la multiplication des cellules cornéennes dans le polymère à l'origine de la biointégration du polymère dans le stroma cornéen.

L'épaisseur du polymère peut être de l'ordre de 0,35 mm et les pores de celui-ci peuvent être compris entre 20 et 100 microns.

Comme cela apparaît sur la figure 1, la kératoprothèse est implantée dans la partie centrale de la cornée, tandis que la partie haptique assure la liaison dans l'épaisseur du stroma cornéen 4.

Après implantation intra-stromale de la partie haptique, les cellules cornéennes colonisent peu à peu le tissu de PTFEe pour assurer un véritable ancrage biologique.

Pour la transplantation, on réalise une cavité périphérique par délamination dans l'épaisseur du stroma 4. Une trépanation centrale permet de recevoir l'optique en silicone 1. Le diamètre de la trépanation est inférieur au diamètre de l'optique, de sorte que la cornée et l'épithélium 12 recouvrent le bord de l'optique 1. Par exemple, si le diamètre hors-tout de la prothèse est de 7 mm, le diamètre de l'optique sera de 4 mm, et le diamètre de la trépanation de 3 mm. Ainsi, le bord de la lentille 1 est inclus dans le stroma ce qui facilite la progression épithéliale sur l'optique.

La surface externe de l'optique 1 est convexe et présente une courbure similaire à celle de la cornée naturelle. La face interne est concave de sorte que l'implant constitue une lentille convergente dont la puissance peut varier entre dix et soixante-dix dioptries en fonction de l'oeil du patient mais est de préférence égale à 43 dioptries pour un patient phake et de 58 dioptries pour un patient aphake. L'épaisseur au centre de la lentille est égale à 0,6 mm, mais peut varier légèrement en fonction de la puissance de l'optique. La même couche de polyvinyle pyrrolidone permet de rendre la prothèse plus agréable à supporter par le patient, facilite l'écoulement des larmes et évite tout dépôt sur la surface de l'optique.

Bien entendu, l'invention n'est pas limitée au mode de réalisation qui vient d'être décrit et représenté. On pourra y apporter de nombreuses modifications de détail sans sortir pour cela du cadre de l'invention.

## Revendications

1. Kératoprothèse comprenant une partie optique transparente (1) formant lentille convergente et une partie haptique (2) de liaison avec le stroma cornéen et constituée par une couronne de polymère colonisable par les cellules cornéennes et surmoulée par son bord interne à la partie optique, caractérisée en ce que la partie haptique est pré-perméabilisée par immersion dans un liquide dont la tension de surface est de l'ordre de 20 dynes/cm, puis par immersion dans un liquide contenant au moins une macro-molécule naturellement présente dans le sang.

2. Kératoprothèse, selon la revendication 1, caractérisée en ce que la macro-molécule présente dans le sang est le sérum du sang du patient.

3. Kératoprothèse, selon la revendication 1, caractérisée en ce que la macro-molécule présente dans le sang du patient est son sang lui-même.

4. Kératoprothèse, selon la revendication 1, caractérisée en ce que l'une des faces au moins de l'optique est revêtue de polyvinyle pyrrolidone.

5. Kératoprothèse, selon la revendication 1, caractérisée en ce que le bord de la lentille est aminci.

6. Outil pour le traitement de la partie haptique de la kératoprothèse selon la revendication 1 et du type comprenant deux branches (5) solidaires l'une de l'autre à l'une de leurs extrémités, les autres extrémités comportant chacune une coupelle (7), chaque coupelle (7) présentant une partie concave, les concavités étant tournées en regard l'une de l'autre, ledit outil pouvant occuper une position d'ouverture dans laquelle les coupelles (7) sont écartées l'une de l'autre, de manière à pouvoir insérer entre celles-ci la lentille, et une position de fermeture dans laquelle le bord des coupelles enserre la partie haptique au voisinage du bord de la lentille, ledit outil étant caractérisé en ce qu'il comporte une vis (9) traversant les branches (5) en un point intermédiaire de leur longueur et recevant un écrou (8).
